# EUROPEAN PATENT APPLICATION

(11) **EP 2 098 193 A2**
(43) Date of publication of application: **09.09.2009**
(21) Application number: 09153373.7
(22) Date of filing: 20.02.2009
(51) Int. Cl.: A61F 2/34

(54) **Prosthesis**

(30) Priority: 28.02.2008 GB 0803676
(71) Applicant: Finsbury (Development) Limited, Randalls Road Leatherhead, Surrey KT22 7BA (GB)
(72) Inventor: Taylor, Andrew Clive, Chichester Surrey PO19 3QQ (GB); Tuke, Michael Anthony, Guildford Surrey GU1 3TF (GB)
(74) Representative: Smaggasgale, Gillian Helen

(57) **Abstract**

An acetabular cup prosthesis comprising a shell component, a liner component located within the shell component and a filling means located in a space between the shell component and the liner component.

## Description

The present invention relates to a prosthesis. More particularly, it relates to a preassembled acetabular cup hip prosthesis.

The efficient functioning of the hip joints is extremely important to the well-being and mobility of the human body. Each hip joint is comprised by the upper portion of the femur which terminates in an offset bony neck surmounted by a ball-headed portion which rotates within the acetabulum in the pelvis. Diseases such as rheumatoid- and osteo-arthritis can cause erosion of the cartlidge lining of the acetabulum so that the ball of the femur and the hip bone rub together causing pain and further erosion. Bone erosion may cause the bones themselves to attempt to compensate for the erosion which may result in the bone becoming misshapen. This misshapen joint may cause pain and may eventually cease to function altogether.

Operations to replace the hip joint with an artificial implant are well-known and widely practiced. Generally, the hip prosthesis will be formed of two components, namely: an acetabular component which lines the acetabulum; and a femoral component which replaces the femoral head. The femoral component may be total femoral head replacement in which case the component includes a head, neck and a stem which in use is inserted into the end of a prepared femur. Alternatively, where appropriate, the femoral head component may be a resurfacing prosthesis which is attached to the head of the femur once it has been suitably machined.

In an operation to insert a prosthetic acetabulum in a patient's pelvis the surgeon first uses a reamer to prepare a cavity of appropriate size in the patient's pelvis. An acetabular cup is then inserted into the cavity. By "appropriate size" is meant a size which is selected by the surgeon as being the most appropriate for that particular patient. Normally, it is desirable to retain as much of the original healthy bone surface as possible.

Commercially available acetabular cups are sold in a range of sizes to suit the needs of individual patients. Generally, acetabular cups are availablein sizes of from 42 mm to 62 mm diameter with 2 mm increments between neighbouring sizes.

There are a number of different types of prosthetic acetabular cups. One type of cup is those made from ultra high molecular weight polyethylene. These are generally cemented into the acetabulum and require only light pressure to seat them in the cement. One alternative cup type has a polyethylene liner unit for articulation with the femur and a metal shell for insertion into the pelvic cavity. These cups may be implanted without cement such that they rely on a jam fit between the metal shell and the patient's acetabulum. Often these metal shells have outer surfaces or coatings which encourage bone to grow into them over time. With this type of prosthesis, the polyethylene liner unit is snapped into the metal shell after the metal shell has been seated in the acetabulum to form the socket part.

More recently, ceramics have been used to form the liner of the acetabulum. In this arrangement, the metal shell, which is generally formed from titanium and which is of a similar thickness to the arrangement in which a polyethylene liner is used, is inserted into the acetabulum. The ceramic liner is then inserted into the shell. It can be difficult for the liner to be accurately aligned in the shell. In addition, this insertion of the liner does require the application of a considerable force which is usually applied by the surgeon using a mallet often via an insertion tool. The force applied by the surgeon on the insertion tool is not controllable and is typically in the region of 5KN but may be of the order of about 35kN. In this connection, it should be noted that the external shape of the liner may be tapered and there will be a co-operating taper in the internal surface of the shell. The considerable force is generally required to lock the taper together.

This force may cause either the outer shell to become deformed and/or the liner to be incorrectly seated in the shell which can lead to various disadvantages. Even if the liner is correctly located and the shell is not deformed during the assembly process, it may become deformed on insertion in the pelvis such that the liner cannot sit properly within the shell such that a space remains between the cup and the liner. Although the presence of this space this does not have an effect on the functioning of the prosthesis, the space may mean that as the femoral head articulates in the acetabular cup prosthesis an acoustic wave can propagate in the space such that a "ringing" sound may be noted by the patient. Whilst the prosthesis provides the patient with an acceptable performance, the sound which is made as they move, leads to patient dissatisfaction and in some cases the sound causes embarrassment.

A further problem is that if the liner is not seated correctly in the shell, in use, forces may not be correctly disipated and the shell may become separated from the liner at one or more points which can also be another cause of ringing.

In vivo, some load of the femur in the acetabulum cup prosthesis can be applied at the edge of the cup due to impingement. This can lead to stripe wear which can induce vibration that may be made audible by the space in the assembly. A further problem with loading at the edge of the cup in a system where a space may be present is that the force exerted through the base of the liner may cause it to become damaged since it is not fully supported by the shell or the patient's pelvis.

It is therefore desirable to provide an acetabular component which offers improved characteristics over known acetabulum cup prosthesis.

Thus, according to the present invention, there is provided an acetabular cup prosthesis comprising a shell component, a liner component located within the shell component and a filling means located in a space between the shell component and the liner component.

The space between the shell component and the liner component will generally only be present in the bowl of the shell component such that there is no space at the rim and the region adjacent thereto.

In a preferred arrangement, the acetabular cup prosthesis is preassembled such that the surgeon inserts the prosthesis as a single unit. This has the benefit that the orientation alignment of the liner in the shell can be controlled at the point of manufacture and the possibility of the errors caused at insertion are removed. In addition, the force applied at the insertion of the liner in the shell can be accurately controlled such that the final prosthesis has the optimum performance. A further advantage is the positioning and, where appropriate, the amount of the filing means can be carefully controlled. A still further benefit is that the prosthesis can be assembled in a sterile environment thereby minimising the risk of debris becoming located between the liner and the shell which is a risk in the arrangements where the prosthesis is assembled in vivo.

The shell component may be formed of any suitable material. It will generally be formed from metal with titanium being particularly preferred. Cobalt/chromium may also be used. The thickness of the shell component will be selected as appropriate. Suitable thicknesses are those of an order of from about 1.5 mm to about 4.5 mm.

The liner component may be formed of any material which has acceptable biocompatibility, hardness and wear resistance. A plastics material such as polyethylene may be used. In one alternative, the liner may be formed from a ceramics material. Suitable ceramic materials include silicon nitride, doped silicon nitride, an alumina-zirconia ceramic, yttria, stabilized zirconia, ceria, stabilized zirconia, zirconia, xirconia ceramics, alumina ceramics, oxonium or mixtures thereof. Ceramic liners may be from about 2 mm to about 5 mm in thickness.

The liner component may be held in position in the shell component as a tight fit, most preferably by the use of a mating taper fit. Alternatively, the liner component may be held in the outer cup component by securement means which are biocompatible and function efficiently. Preferred securement means include cement or screws. The securement means may be formed on the inner surface of the shell component, on the outer surface of the liner component or on both the cup component and the and liner component such that the liner is a snap-fit in the shell component.

The filling means may be in any suitable form. In one arrangement, the filling means is an acoustic damping means. That is to say it acts in use to reduce or remove sounds such as the "ringing" noted in prior art arrangements. In one arrangement, the filling means is formed from material which is liquid throughout the life of the prosthesis or in one alternative arrangement, it may be formed from material which is liquid while the prosthesis is being manufactured but which subsequently becomes solid. In a still further arrangement the fillingmaterial may be a solid. The solid material may be rigid in at least compression but in an alternative, preferred arrangement may have at least some resilience when compressed.

Although it is anticipated that the filling material will not come into contact with the body, it may be appropriate to select the filling material from biocompatible material.

Examples of filling materials which remain liquid include suitable silicones.

Where the filling material is to be formed from material which is liquid during manufacture but which subsequently becomes solid, any suitable material may be used. Suitable materials include acrylics and expoxy resins may be used.

Similarly if a solid material is to be used, any suitable material may be selected. The solid material may be, for example, formed from silicone. In general compliant materials such as rubber or compliant plastics may be used.

The filling means may fill the whole of the space between the shell component and the liner component. In an alternative arrangement, the filling material may only fill a proportion of the space. For example, the filling material may be in the form of a silicone ring. In one arrangement, the space may be filled completely in the thickness of the space but not in the radial direction.

## Claims

1. An acetabular cup prosthesis comprising a shell component, a liner component located within the shell component and a filling means located in a space between the shell component and the liner component.

2. An acetabular cup prosthesis according to Claim 1 wherein the prosthesis is preassembled.

3. An acetabular cup prosthesis according to Claim 1 or 2 wherein the shell component is formed from titanium.

4. An acetabular cup prostheses according to Claim 3 wherein the shell component has a thickness of from about 1mm to about 3mm,

5. An acetabular cup prosthesis according to any one of Claims 1 to 4 wherein the liner is formed from a ceramics material.

6. An acetabular cup prosthesis according to Claim 5 wherein the ceramic material is selected from silicon nitride, doped silicon nitride, an alumina-zirconia ceramic, yttria, stabilized zirconia, ceria, stabilized zirconia, zirconia, xirconia ceramics, alumina ceramics, oxonium and mixtures thereof.

7. An acetabular cup prosthesis according to any one of Claims 1 to 6 wherein the filling means is an acoustic damping means.

8. An acetabular cup prosthesis according to any one of Claims 1 to 7 wherein the filling means is formed from material which is liquid throughout the life of the prosthesis.

9. An acetabular cup prosthesis according to any one of Claims 1 to 7 wherein the filling means is formed from material which is liquid while the prosthesis is being manufactured but which becomes solid.

10. An acetabular cup prosthesis according to Claim 9 wherein the material is hydroxyapatite.

11. An acetabular cup prosthesis according to any one of Claims 1 to 7 wherein the filling means is formed from a solid material.

12. An acetabular cup prosthesis according to Claim 11 wherein the material is silicone.

13. An acetabular cup prosthesis according to any one of Claims 1 to 12 wherein the filling means fills the whole of the space between the shell component and the liner component.

14. An acetabular cup prosthesis according to any one of Claims 1 to 12 wherein the filling means fill a proportion of the whole of the space between the shell component and the liner component.

15. An acetabular cup prosthesis according to Claim 14 wherein the fillingmeans is a silicone ring.
